(19) Europäisches Patentamt
European Patent Office
Office européen des brevets

(11) Veröffentlichungsnummer: **0 027 610**
**B1**

(12) # EUROPÄISCHE PATENTSCHRIFT

(45) Veröffentlichungstag der Patentschrift: **16.06.82**

(51) Int. Cl.³: **C 07 C 125/073,** C 07 C 125/077

(21) Anmeldenummer: **80106162.3**

(22) Anmeldetag: **10.10.80**

(54) **Verfahren zur Herstellung von Methylen-bis-phenylcarbaminsäureestern und Polymethylen-polyphenylcarbaminsäureestern.**

(30) Priorität: **18.10.79 DE 2942137**

(43) Veröffentlichungstag der Anmeldung: **29.04.81 Patentblatt 81/17**

(45) Bekanntmachung des Hinweises auf die Patenterteilung: **16.06.82 Patentblatt 82/24**

(84) Benannte Vertragsstaaten: **BE DE FR GB IT NL**

(56) Entgegenhaltungen:
**DE-A-2 832 379**
**US-A-2 946 768**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Rubensstrasse 25, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von Methylen-bis-phenylcarbaminsäureestern und Polymethylen-polyphenylcarbaminsäureestern

Die Erfindung betrifft ein neues Verfahren zur Herstellung von Methylen-bis-phenylcarbaminsäureestern und davon abgeleiteten höheren homologen Polymethylen-polyphenylcarbaminsäureestern durch Umsetzung von N-Phenylcarbaminsäureestern mit Formaldehyd oder formaldehydabspaltenden Verbindungen in Gegenwart von Säuren und von Carbonsäuren.

Methylen-bis-phenylcarbaminsäureester und davon abgeleitete höhere Homologe sind wertvolle Ausgangsprodukte für die Herstellung von Methylen-bis-phenylisocyanaten und den entsprechenden Polymethylenpolyphenylisocyanaten, die bekanntlich für die Herstellung von Polyurethanen Verwendung finden (DE-OS 2635490). Die im Handel erhältlichen derartigen Isocyanate werden allgemein durch Phosgenierung der bei der Kondensation von Anilin mit Formaldehyd in Gegenwart von wässrigen Säuren erhältlichen Amine hergestellt.

Die Herstellung der Methylen-bis-phenylcarbaminsäureester erfolgt durch Umsetzung derartiger Anilin-Formaldehyd-Kondensationsprodukte mit Chlorameisensäureestern in Gegenwart von Basen oder durch Umsetzung der entsprechenden Isocyanate mit Alkoholen.

Diese Verfahren haben den Nachteil, dass die Umsetzung mit dem stark toxischen Phosgen aus Sicherheitsgründen eine aufwendige Technik erfordert und die Abtrennung der bei den einzelnen Umsetzungen einerseits benötigten und andererseits als Nebenprodukt anfallenden Säuren die Umwelt stark belastet.

Methylen-bis-phenylcarbaminsäureester lassen sich auch durch Umsetzung von Methylen-bis-nitrophenyl mit Alkoholen und Kohlenmonoxid herstellen (s. DE-AS 1568044). Da die Darstellung der hierfür benötigten Nitroverbindungen grosse Schwierigkeiten bereitet, hat dieses Verfahren keine technische Bedeutung erlangt.

In der US-PS 2946768 wird ein Verfahren beschrieben, bei dem man Phenylcarbaminsäureester mit Formaldehyd oder Formaldehyd abgebenden Verbindungen in Gegenwart von Säuren erhitzt. Über die dabei erhaltenen Kondensationsprodukte wird gesagt, dass sie C-C-Verknüpfungen aufweisen, wobei aber auch N-C-Verknüpfungen nicht ausgeschlossen werden (s. Spalte 2, Zeilen 65-70).

In der DE-OS 2832379 wird angegeben, dass mit früher nicht bekannten analytischen Methoden nachgewiesen werden konnte, dass bei dem Verfahren der US-PS 2946768 Formaldehyd zur Reaktion am Stickstoffatom des Carbaminsäureesters neigt und daher 15 bis 50 Gew.-% an unerwünschten N-C-verknüpften Produkten entstehen. Durch ein analog Beispiel 2 der US-PS 2946768 durchgeführtes Vergleichsbeispiel (s. Beispiel 1) wird dieses Ergebnis bestätigt. Ausserdem werden noch andere Nebenprodukte gebildet, beispielsweise entstehen durch saure Hydrolyse des Carbaminsäureesters Amine.

Da es kein Verfahren zur Abtrennung der über das Stickstoffatom verknüpften Produkte, die bei der Pyrolyse keine Isocyanate liefern, gibt, sind die nach der US-PS 2946768 hergestellten Reaktionsgemische zur Herstellung von Isocyanaten ungeeignet. Dieses Verfahren ist daher in technischer und ökonomischer Hinsicht unbefriedigend.

In der DE-OS 28 32 379 wird nun ein Verfahren zur Umlagerung dieser unerwünschten Nebenprodukte zu Methylen-bis-phenylcarbaminsäureestern und davon abgeleiteten höheren homologen Polymethylenpolyphenylcarbaminsäureestern beschrieben. Es besteht darin, dass man die nach dem Verfahren der US-PS 2946768 hergestellten Reaktionsgemische unter praktisch wasserfreien Bedingungen bei 50 bis 170°C mit starken Protonensäuren oder Lewissäuren umsetzt. Bei dieser zweistufigen Arbeitsweise wird zunächst aus Phenylcarbaminsäureestern und Formalin in Gegenwart erheblicher Mengen wässriger Säuren ein Kondensationsprodukt hergestellt, welches von der Säure befreit und getrocknet werden muss, bevor es schliesslich unter wasserfreien Bedingungen erneut mit grossen Mengen an Säure, die nach Ablauf der Reaktion ebenfalls wieder vollständig zu entfernen sind, umgesetzt wird. Die Abwasserbelastung durch die grossen Säuremengen und die teilweise gebildeten Nebenprodukte, z.B. durch die bei der sauren Hydrolyse entstandenen Amine, ist ein ernstes Problem.

Es wurde nun gefunden, dass man bei der Herstellung von Methylen-bis-phenylcarbaminsäureestern und Polymethylen-polyphenylcarbaminsäureestern durch Umsetzung von N-Phenylcarbaminsäureestern mit Formaldehyd oder Formaldehyd abgebenden Verbindungen in Gegenwart einer starken Säure die genannten Nachteile vermeidet, wenn man die Umsetzung in einer Carbonsäure vornimmt.

Nach dem neuen Verfahren werden die gewünschten Methylen-bis-phenylcarbaminsäureester und Polymethylen-polyphenylcarbaminsäureester besonders einfach in guter Ausbeute und hoher Reinheit in einer einzigen Stufe erhalten. Dass man bei der erfindungsgemässen Umsetzung keine C-N-verknüpften Kondensationsprodukte erhält, ist überraschend, da diese Nebenprodukte z.B. dann entstehen, wenn man die säurekatalysierte Kondensation von Carbaminsäureestern mit Formaldehyd in den üblichen organischen Lösungsmitteln, wie Chlorbenzol, Nitrobenzol oder Toluol, durchführt (s. Vergleichsbeispiele 2 und 3). Überraschend ist ausserdem, dass keine Verseifung des Carbaminesters zu beobachten ist, obwohl aus der Literatur bekannt ist, dass der Zusatz von Essigsäure die Hydrolyse des Carbaminsäureesters mitunter erleichtert (Houben-Weyl Bd. 11/1, S. 948).

Das Reaktionsschema kann für den Fall der Bildung des Methylen-bis-(4-phenylcarbaminsäu-

reesters) aus dem N-Phenylcarbaminsäuremethylester und Formaldehyd durch folgende Formeln wiedergegeben werden.

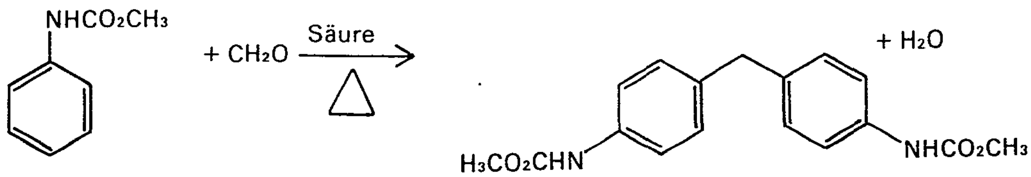


Bei dem erfindungsgemässen Verfahren werden gleichzeitig auch höhere homologe Polymethylen-polyphenylcarbaminsäureester, das sind 3- und mehrere durch Methylenbrücken miteinander verbundene Benzolringe enthaltende Carbaminsäureester, gebildet, da Formaldehyd in untergeordnetem Masse auch mit bereits gebildetem Methylen-bis-phenylcarbaminsäureester reagiert.

Als N-Phenylcarbaminsäureester kommen z.B. Verbindungen der Formel

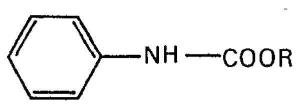

in Betracht, in der R ein Alkylrest mit 1 bis 3 C-Atomen bedeutet und der Phenylrest in den o- und/oder m-Stellungen beispielsweise durch Methyl- oder Methoxygruppen oder durch Halogenatome wie Chlor- oder Bromatome, substituiert sein kann.

Geeignete N-Phenylcarbaminsäureester sind beispielsweise N-Phenylcarbaminsäuremethyl-, äthyl- oder propylester, N-o-Tolylcarbaminsäuremethyl- oder äthylester, N-2,6-Dimethylphenylcarbaminsäuremethylester oder N-o-Chlorphenylcarbaminsäureäthylester.

Anstelle von Formaldehyd können auch Formaldehyd abspaltende Verbindungen verwendet werden, wie Paraformaldehyd oder Trioxan.

Das Molverhältnis Formaldehyd:Carbaminsäureester liegt zweckmässig bei 1:0,5 bis 1:10, vorzugsweise bei 1:1,5 bis 1:3. Sollen jedoch hauptsächlich Methylen-bis-phenylcarbaminsäureester hergestellt werden, so verwendet man vorzugsweise ein Verhältnis von 1:4 bis 1:8.

Die als Katalysatoren wirkenden Säuren, die man z.B. in Mengen von 1 bis 100, vorzugsweise 10 bis 60 Mol.%, bezogen auf den Carbaminsäureester, anwendet, sind starke Säuren. Beispielsweise seien Phosphorsäure, Schwefelsäure, Chlorwasserstoff, Alkylsulfonsäure wie Methansulfonsäure oder Arylsulfonsäure wie p-Toluolsulfonsäure genannt. Entsprechend einer besonders vorteilhaften Ausführungsform der Erfindung verwendet man eine starke Säure, die vom Reaktionsgemisch destillativ abgetrennt werden kann, beispielsweise Trifluormethansulfonsäure. Auf diese Weise entfällt die Aufarbeitung des Reaktionsgemisches mit Wasser oder Basen und die Säure kann direkt erneut der Reaktion zugeführt werden.

Nach einer weiteren vorteilhaften Ausführungsform der Erfindung verwendet man als Säuren stark saure organische Kationenaustauscher, beispielsweise sulfonsaure Austauschharze. Diese Ionenaustauscher werden nach an sich bekannten Methoden entweder im Reaktionsgemisch suspendiert oder in einem Festbett angeordnet. Die aufwendige Abtrennung des sauren Katalysators mit Wasser oder Basen entfällt hierbei.

Als Carbonsäuren kommen sowohl substituierte als auch unsubstituierte Carbonsäuren, wie aliphatische Carbonsäuren mit 1 bis 6 C-Atomen in Betracht. Besonders geeignet sind Essigsäure, Propionsäure, Ameisensäure oder Chloressigsäure. Man wendet sie in einer Menge von 50 bis 500 g, vorzugsweise 100 bis 200 g pro Mol Carbaminsäureester an.

Man führt die erfindungsgemässe Umsetzung z.B. bei Temperaturen von 50 bis 160°C, vorzugsweise 90 bis 140°C durch. Die Umsetzung, die etwa nach 0,5 bis 10 Stunden beendet ist, wird im allgemeinen so durchgeführt, dass man entweder zu einem Gemisch aus dem Carbaminsäureester und dem Katalysator unter Rühren bei der Reaktionstemperatur beispielsweise ein Gemisch aus Paraformaldehyd und der Carbonsäure langsam zugibt oder dass man beispielsweise ein Gemisch aus Carbaminsäureester, Carbonsäure, Parformaldehyd und Katalysator unter Rühren erhitzt und die Gemische die entsprechende Zeit auf der Reaktionstemperatur hält. Die Isolierung des Reaktionsproduktes erfolgt nach herkömmlichen Methoden, z.B. durch destillative Abtrennung der Carbonsäure und Extraktion des Katalysators mit Wasser oder Neutralisation mit einer Base. Das gegebenenfalls vorhandene unumgesetzte Ausgangsprodukt wird mittels einer Vakuumdestillation abgetrennt.

Man kann das neue Verfahren in Einzelansätzen oder kontinuierlich durchführen.

Beispiel 1 (Vergleichsbeispiel)

Analog Beispiel 2 der DE-PS 1 042 891 wird ein Gemisch aus 183 Teilen Phenylcarbaminsäuremethylester, 500 ml Wasser und 86 Teilen Formalin (30%ig) unter Rühren auf 100°C erwärmt, dann gibt man 100 ml konzentrierte Salzsäure hinzu. Das Reaktionsgemisch wird anschliessend 20 Stunden bei 100°C gerührt. Nach Beendigung der Umsetzung wird die wässrige Phase abgetrennt und das Reaktionsprodukt dreimal mit heissem Wasser gewaschen. Anschliessend wird unumgesetztes Ausgangsprodukt im Vakuum abdestilliert. Der Rückstand wird mittels Hochdruckflüssigkeits-Chromatographie (HPLC) analysiert. Er enthält 50% Methylen-bis-phenylcarbaminsäuremethylester, 9% Dreikernprodukt, 16% N-C-verknüpftes Zweikernprodukt und 10% N-C-verknüpftes Dreikernprodukt. Der Rest besteht aus nicht näher identifizierten höherkernigen Verbindungen.

Beispiel 2 (Vergleichsbeispiel)

Ein Gemisch aus 90 Teilen Phenylcarbaminsäuremethylester, 250 ml Chlorbenzol und 40 Teilen Formalin (30%ig) wird unter Rühren auf 100°C erwärmt, dann gibt man 50 ml konzentrierte Salzsäure hinzu. Das Reaktionsgemisch wird anschliessend 20 Stunden bei 100°C gerührt. Nach Beendigung der Umsetzung wird die wässrige Phase abgetrennt, mit Wasser zweimal gewaschen und anschliessend Chlorbenzol und unumgesetztes Ausgangsprodukt abdestilliert. Entsprechend der HPLC-Analyse enthält der Rückstand 49% Methylen-bis-phenylcarbaminsäuremethylester, 12% Dreikernprodukt, 15% N-C-verknüpftes Zweikern- und 9% Dreikernprodukt. Der Rest besteht aus nicht näher identifizierten höherkernigen Verbindungen.

Beispiel 3 (Vergleichsbeispiel)

In einem Rührreaktor wird ein Gemisch aus 151 Teilen Phenylcarbaminsäuremethylester, 15 Teilen Paraformaldehyd, 100 Teilen Nitrobenzol und 30 Teilen Lewatit SPC-108 unter Rühren auf 110°C erhitzt und 10 Stunden bei dieser Temperatur gerührt. Nach Beendigung der Umsetzung wird der Katalysator abgetrennt und im Vakuum Nitrobenzol und nicht umgesetztes Ausgangsmaterial abdestilliert. Man erhält 112 Teile eines Destillationsrückstandes, der entsprechend der HPLC-Analyse aus 46% Methylen-bis-phenylcarbaminsäuremethylester, 21% Dreikernprodukt und 13% N-C-verknüpfte Zweikern- und Dreikernprodukt besteht. Der Rest besteht aus höherkernigen Produkten.

«Lewatit SPC-108» ist ein im Handel erhältlicher makroporöser sulfonsaurer Kationenaustauscher aus Styrol-Divinylbenzol (8%)-Copolymerisat, Korngrösse 0,3–1 mm, Totalkapazität ca. 4,2 mVal/g Trockensubstanz.

Beispiel 4

In einem Rührreaktor wird ein Gemisch aus 151 Teilen Phenylcarbaminsäuremethylester, 15 Teilen Paraformaldehyd, 100 Teilen Essigsäure und 30 Teilen Lewatit SPC-108 unter Rühren auf 110°C erhitzt und 10 Stunden bei dieser Temperatur gerührt. Nach Beendigung der Umsetzung wird der Katalysator abgetrennt und im Vakuum das Lösungsmittel und nicht umgesetztes Ausgangsmaterial abdestilliert. Man erhält 149 Teile eines Destillationsrückstandes, der entsprechend der HPLC-Analyse aus 55 Teilen Methylen-bis-phenylcarbaminsäuremethylester, 29% Dreikernprodukt und 16% höherkernigen Produkten besteht.

Beispiel 5

In einem Rührreaktor wird ein Gemisch aus 151 Teilen Phenylcarbaminsäuremethylester, 15 Teilen Trioxan, 150 Teilen Chloressigsäure und 50 Teilen Lewasorb AC-10 unter Rühren auf 110°C erhitzt und 10 Stunden bei dieser Temperatur gerührt. Nach Beendigung der Reaktion wird der Katalysator abgetrennt und im Vakuum nicht umgesetztes Ausgangsmaterial abdestilliert. Man erhält 145 Teile eines Destillationsrückstandes, der entsprechend der HPLC-Analyse aus 53% Methylen-bis-phenylcarbaminsäuremethylester, 30% Dreikernprodukt und 17% höherkernigen Kondensationsprodukten besteht.

Lewasorb AC-10 ist ein im Handel erhältlicher gelartiger sulfonsaurer Kationenaustauscher aus Styrol-Divinylbenzol (8%)-Copolymerisat, Korngrösse 0,01 bis 0,2 mm, Totalkapazität ca. 4,2 mVal/g Trockensubstanz.

Beispiel 6

In einem Rührreaktor wird ein Gemisch aus 165 Teilen Phenylcarbaminsäureäthylester, 10 Teilen 50%igem wässrigen Formaldehyd, 100 Teilen Essigsäure und 20 Teilen Schwefelsäure unter Rühren auf 100°C erhitzt und 10 Stunden bei dieser Temperatur gehalten. Nach Beendigung der Reaktion wird im Vakuum nicht umgesetztes Ausgangsprodukt und Essigsäure abdestilliert. Der Destillationsrückstand wird mit Wasser behandelt und in Toluol umkristallisiert. Man erhält 46 Teile Methylen-bis-(4-phenylcarbaminsäureäthylester).

**Patentansprüche**

1. Verfahren zur Herstellung von Methylen-bis-phenylcarbaminsäureestern und Polymethylenpolyphenylcarbaminsäureestern durch Umsetzung von N-Phenylcarbaminsäureestern mit Formaldehyd oder Formaldehyd abgebenden Verbindungen in Gegenwart einer starken Säure, dadurch gekennzeichnet, dass man die Umsetzung in einer Carbonsäure vornimmt.

2. Verfahren nach Anspruch 1, dadurch gekennzeichnet, dass man die Umsetzung bei 50 bis 160°C durchführt.

3. Verfahren nach Anspruch 1, dadurch gekennzeichnet, dass man als Carbonsäure Essigsäure, Propionsäure oder Chloressigsäure verwendet.

4. Verfahren nach Anspruch 1, dadurch gekennzeichnet, dass man als starke Säuren stark saure organische Kationenaustauscher verwendet.

**Claims**

1. A process for the preparation of methylene-bis-(phenylcarbamic acid esters) and polymethylene-poly-(phenylcarbamic acid esters) by reacting N-phenylcarbamates with formaldehyde or formaldehyde donors in the presence of a strong acid, characterized in that the reaction is carried out in a carboxylic acid.

2. A process as claimed in claim 1, characterized in that the reaction is carried out at from 50° to 160°C.

3. A process as claimed in claim 1, characterized in that the carboxylic acid used is acetic acid, propionic acid or chloroacetic acid.

4. A process as claimed in claim 1, characterized in that the strong acids used are strongly acidic organic cation exchangers.

## Revendications

1. Procédé pour la préparation d'esters d'acide méthylène-bis-phénylcarbamique et d'esters d'acides polyméthylène-polyphénylcarbamiques par réaction d'esters d'acide N-phénylcarbamique avec le formaldéhyde ou des composés donnant du formaldéhyde par dissociation, en présence d'un acide fort, caractérisé en ce que la réaction est menée dans un acide carboxylique.

2. Procédé selon la revendication 1, caractérisé en ce qu'on effectue la réaction à une température de 50 à 160°C.

3. Procédé selon la revendication 1, caractérisé en ce qu'on utilise, comme acide carboxylique, l'acide acétique, l'acide propionique ou l'acide chloracétique.

4. Procédé selon la revendication 1, caractérisé en ce qu'on utilise, comme acides forts, des échangeurs de cations organiques fortement acides.